**Europäisches Patentamt**

**European Patent Office**

**Office européen des brevets**

(19)

(11) Numéro de publication : **0 168 095**
**B1**

(12)

# FASCICULE DE BREVET EUROPÉEN

(45) Date de publication du fascicule du brevet :
**20.01.88**

(21) Numéro de dépôt : **85201004.0**

(22) Date de dépôt : **25.06.85**

(51) Int. Cl.⁴ : **C 12 P 7/62**, C 08 G 63/72

(54) **Procédé pour l'extraction de poly-beta-hydroxybutyrates au moyen d'un solvant à partir d'une suspension aqueuse de microorganismes.**

(30) Priorité : **06.07.84 FR 8410921**

(43) Date de publication de la demande :
**15.01.86 Bulletin 86/03**

(45) Mention de la délivrance du brevet :
**20.01.88 Bulletin 88/03**

(84) Etats contractants désignés :
**AT BE CH DE FR GB IT LI LU NL SE**

(56) Documents cités :
**EP-A- 0 014 490**
**EP-A- 0 036 699**
**EP-A- 0 124 309**

(73) Titulaire : **SOLVAY & Cie (Société Anonyme)**
**Rue du Prince Albert, 33**
**B-1050 Bruxelles (BE)**

(72) Inventeur : **Vanlautem, Noel**
**Rue des Drapiers, 17**
**B-1300 Wavre (BE)**
Inventeur : **Gilain, Jacques**
**Avenue de la Ramée, 10**
**B-1180 Bruxelles (BE)**

Jouve, 18, rue St-Denis, 75001 Paris, France

## Description

La présente invention concerne un procédé pour l'extraction de poly-bêta-hydroxybutyrates à partir d'une suspension aqueuse de microorganismes dans lequel on réalise une distillation azéotropique couplée à une extraction par solvant.

De nombreux microorganismes sont capables de synthétiser des poly-bêta-hydroxybutyrates dont la fonction principale dans ces microorganismes semble être le stockage de l'énergie sous forme de matière carbonée. Ces poly-bêta-hydroxybutyrates (PHB) constituent une matière première intéressante sur le plan industriel. Diverses techniques ont déjà été envisagées pour les séparer de la biomasse.

Ainsi on a déjà proposé dans la demande de brevet EP-A 0 015 123 de sécher les cellules bactériennes par introduction d'un gaz chaud dans une première étape puis après refroidissement dans une deuxième étape d'extraire les poly-bêta-hydroxybutyrates avec un solvant adéquat. A ce procédé en deux étapes on peut additionner des étapes supplémentaires telles que notamment une étape dans laquelle les cellules bactériennes sont lysées avant l'extraction avec le solvant.

Un autre procédé est décrit dans la demande de brevet EP-A 0 046 017. Selon ce document les cellules bactériennes sont floculées à température élevée par modification du pH dans une première étape, puis dans une seconde étape après refroidissement la suspension des cellules bactériennes floculées est chauffée par introduction de vapeur d'eau sous pression ; cette opération est suivie ou précédée d'une modification supplémentaire du pH. Les cellules floculées sont ensuite séchées de manière à obtenir un produit granulaire poreux en vue du contact ultérieur avec le solvant pour l'extraction du PHB.

Dans la demande de brevet EP-A 0 058 480, on décrit un autre procédé nécessitant plusieurs étapes. Selon ce procédé, la culture bactérienne est d'abord séchée par atomatisation à température élevée, ensuite les cellules séchées sont mises en contact après refroidissement avec du méthanol en vue d'extraire les lipides et enfin avec un solvant en vue d'extraire les poly-bêta-hydroxybutyrates. La solution est ensuite refroidie davantage en dessous de 0 °C, puis chauffée à 20 °C durant 30 minutes, un gel est ainsi obtenu. Ce gel est pressé en vue d'expulser le solvant.

Dans le brevet EP-A-0 014 490 on décrit par ailleurs un procédé de séparation de poly-bêta-hydroxybutyrates d'une biomasse par extraction au moyen de solvants halogénés liquides tels que les chloroéthanes et les chloropropanes. Dans ce procédé, la biomasse peut être traitée telle quelle par le solvant d'extraction, mais les exécutions exemplifiées décrivent le traitement de la biomasse séparée de son milieu de culture et séchée.

Les procédés de l'art antérieur présentent toutefois des inconvénients lorsqu'ils sont mis en œuvre dans des installations industrielles du fait de la complexité imposée aux installations et de la dépense énergétique élevée due aux chauffages et refroidissements successifs.

La présente invention vise à fournir un procédé d'extraction de poly-bêta-hydroxybutyrates, ne présentant plus les inconvénients des procédé antérieurs.

La présente invention concerne à cet effet un procédé d'extraction de poly-bêta-hydroxybutyrates au moyen d'un solvant à partir d'une suspension aqueuse de microorganismes caractérisé en ce que l'on

a) choisit un solvant des poly-bêta-hydroxybutyrates formant un azéotrope à minimum avec l'eau.
b) élimine l'eau par distillation azéotropique
c) réalise l'extraction au moins partiellement à la température d'ébullition de l'azéotrope.

Par solvant formant un azéotrope à minimum avec l'eau, la demanderesse entend un solvant qui lorsqu'il est mélangé à l'eau, forme un mélange dont la température d'ébullition est constante et est inférieure à la température d'ébullition du solvant ou de l'eau pris séparément et qui garde une composition constante à une pression donnée.

[Encyclopédie Internationale des Sciences et des Techniques — Larousse (1970, Vol. 2, p. 205-206)].

Comme solvant des poly-bêta-hydroxybutyrates formant un azéotrope à minimum avec l'eau on peut utiliser tout solvant ayant cette propriété. Habituellement les solvants sont choisis parmi les solvants organiques et plus particulièrement parmi les solvants aliphatiques halogénés. Parmi ceux-ci on travaille généralement avec des solvants aliphtiques saturés chlorés.

De bons résultats ont été obtenus avec les chlorométhanes, les chloroéthanes et les chloropropanes. Parmi ceux-ci on préfère travailler avec le 1,2-dichloroéthane, 1,1,2-trichloroéthane et le 1,2-dichloropropane.

Tout particulièrement préféré comme solvant des poly-bêta-hydroxybutyrates formant un azéotrope avec l'eau est le 1,2-dichloroéthane.

A titre informatif les points d'ébullition de l'azéotrope à minimum, à pression atmosphérique, que ces solvants forment avec l'eau sont repris au Tableau ci-après.

# 0 168 095

Tableau

| Solvant | Point d'ébullition °C (P = 1,0 bar) | Azéotrope avec eau °C |
|---|---|---|
| 1,1,2-trichloroéthane | 113,7 | 86,0 |
| 1,2-dichloroéthane | 83 | 72,0 |
| 1,2-dichloropropane | 96 | 78 |

Les suspensions aqueuses de microorganismes que l'on peut traiter selon l'invention peuvent être obtenues à partir de microorganismes d'origines diverses. La sélection des microorganismes se fait en général sur la base de la quantité relative de poly-bêta-hydroxybutyrates contenue dans le microorganisme ainsi qu'en fonction de la vitesse de croissance du microorganisme et de sa vitesse de synthèse des poly-bêta-hydroxybutyrates.

Les microorganismes peuvent être traités directement par le solvant d'extraction dans leur milieu de culture. Il est cependant également possible d'utiliser des suspensions aqueuses de microorganismes résultant d'une séparation partielle des microorganismes de leur milieu de culture préalablement à l'extraction. Cette séparation partielle peut être effectuée par tous les moyens connus à cet effet. La culture peut être centrifugée ou ultrafiltrée en vue d'être concentrée, ceci peut être suivi ou non d'un ou de plusieurs lavages en vue d'éliminer une fraction des résidus du milieu nutritif. Le procédé de l'invention s'applique également à des suspensions aqueuses de microorganismes provenant de la remise en suspension dans l'eau de cultures préséchées. Il est toutefois évident qu'une telle technique n'est pas préférée du fait de la dépense d'énergie supplémentaire qu'elle entraîne.

Le rapport entre la quantité de la suspension aqueuse de microorganismes mise en œuvre et le solvant d'extraction utilisé n'est pas critique en soi. On travaille en général avec des rapports en poids compris entre 1 : 1 et 1 : 100. Habituellement on emploie des rapports compris entre 1 : 2 et 1 : 50 et enfin on opère de préférence avec des rapports compris entre 1 : 5 et 1 : 20.

Le choix de ce rapport est influencé par divers paramètres tels que la nature des microorganismes à traiter, la température, le nombre d'extractions et le rendement souhaité par extraction. En outre, l'élimination des résidus cellulaires est d'autant plus facile qu'il y a moins de microorganismes dans le milieu d'extraction.

La pression à laquelle est effectué le procédé n'est pas critique et est généralement comprise entre 0,1 et 10 bar.

Dans le procédé de l'invention, l'eau est éliminée par séchage azéotropique. L'élimination de l'eau a lieu jusqu'à l'obtention d'une phase d'un solvant anhydre ou d'un solvant encore saturé en eau ; ce qui se traduit par l'obtention d'une seule phase liquide limpide.

Le procédé peut être réalisé dans tout appareillage conçu à cet effet. On peut réaliser le procédé selon l'invention en continu ou en discontinu avec les suspensions aqueuses de microorganismes et des solvants d'extraction circulant dans le même sens ou dans des sens opposés.

Dans le procédé selon l'invention le solvant saturé ou non en eau résultant de la distillation azéotropique peut être éventuellement recyclé comme solvant d'extraction.

Après extraction le solvant contenant les poly-bêta-hydroxybutyrates peut être débarrassé des composés non solubles dans le milieu tels que les membranes cellulaires de microorganismes. Pour ce faire on peut utiliser n'importe quel moyen connu. Habituellement cette opération est effectuée par une ou plusieurs filtrations.

Les poly-bêta-hydroxybutyrates extraits peuvent être séparés du solvant d'extraction par n'importe quelle méthode connue à cet effet. Ainsi, on peut les séparer par évaporation du solvant ou encore par simple addition d'un agent précipitant. Comme agents précipitants utilisables on peut citer les composés non solvants des poly-bêta-hydroxybutyrates tels que l'éther de pétrole, les hydrocarbures aliphatiques non substitués, les composés aromatiques dont le benzène ou encore les alcools aliphatiques. Des agents précipitants préférés sont les alcools et cétones aliphatiques et plus particulièrement pour des raisons économiques, le méthanol et l'éthanol.

Les poly-bêta-hydroxybutyrates séparés du solvant d'extraction peuvent être ensuite purifiés par un ou plusieurs lavages avec des non solvants tels que ceux cités ci-avant et être séchés, après quoi ils se présentent sous la forme d'une masse blanche. Les opérations de récupération et de purification se font habituellement à température ambiante.

Les poly-bêta-hydroxybutyrates sont des polymères ayant de nombreuses applications et notamment en chirurgie où ils peuvent être utilisés sous forme de fils car ils sont aisément stérilisables. En outre, ces

3

polymères peuvent être mis en forme par les différentes techniques connues de moulage pour faire des prothèses. On peut encore les filer ou les extruder suivant les méthodes habituelles. Enfin, ces poly-bêta-hydroxybutyrates peuvent être dépolymérisés sous forme d'oligomères ou même sous forme de monomère qui est l'acide bêta-hydroxybutyrique. Pour ce faire on peut notamment utiliser des méthodes telles que celle décrite dans la demande de brevet FR-A-2 486 072.

Les exemples suivants servent à illustrer l'invention.

## Exemple 1

Dans un ballon de 2 l. équipé d'un agitateur, d'un thermomètre, d'une ampoule et d'un condenseur à eau, on introduit 79,5 g d'une suspension aqueuse de microorganismes constituée d'une culture bactérienne de la souche Alcaligenes eutrophus contenant 53 g de matière sèche.

On introduit ensuite 1 l de 1,2-dichloroéthane. Sous bonne agitation on porte la suspension à reflux, à pression atmosphérique, en 0,4 h. A 72 °C l'azéotrope à minimum eau-1,2-dichloroéthane est éliminé en 1,5 h. Le solvant organique est recyclé lors de cette distillation.

Quand la totalité de l'eau a été éliminée sous forme d'azéotrope, la température atteint 83 °C et cette température est maintenue durant 0,5 h.

La suspension est ensuite rapidement refroidie puis filtrée sous pression à 3 bar afin d'éliminer les restes solides des microorganismes. Le gâteau est lavé avec 300 ml de 1,2-dichloroéthane.

La solution organique est ensuite évaporée à sec et 29,15 g de poly-bêta-hydroxybutyrates sont récupérés.

Le rendement de l'extraction est donné par le poids du polymère extrait sur le poids de matière sèche des microorganismes mis en œuvre et s'élève à 55 %.

## Exemples 2 et 3

On répète l'exemple 1 en mettant en œuvre 106 g d'une suspension aqueuse de microorganismes contenant 53 g de matière sèche.

L'entraînement azéotropique de l'eau, à 72 °C, dure 1,6 h.

Ensuite, la température de 83 °C est maintenue dans un premier cas (exemple 2) durant 0,4 h et dans un second cas (exemple 3) durant 1,4 h.

On récupère respectivement 28,6 g (exemple 2) et 30,0 g (exemple 3) de poly-bêta-hydroxybutyrates [ce qui correspond à des rendements d'extraction de respectivement 54 % (exemple 2) et 56 % (exemple 3)].

On peut déduire de ces résultats que l'invention permet d'obtenir des rendements d'extraction optimaux des poly-bêta-hydroxybutyrates par un procédé intégré couplant une opération de séchage par azéotropie à une opération d'extraction proprement dite.

## Revendications

1. Procédé pour l'extraction de poly-bêta-hydroxybutyrates au moyen d'un solvant à partir d'une suspension aqueuse de microorganismes caractérisé en ce que l'on :
   a) choisit un solvant de poly-bêta-hydroxybutyrate formant un azéotrope à minimum avec l'eau.
   b) élimine l'eau par distillation azéotropique, et
   c) réalise l'extraction au moins partiellement à la température d'ébullition de l'azéotrope.

2. Procédé selon la revendication 1 caractérisé en ce que l'on utilise comme solvant des poly-bêta-hydroxybutyrates formant un azéotrope à minimum avec l'eau, un solvant aliphatique halogéné.

3. Procédé selon les revendications 1 et 2 caractérisé en ce que le solvant des poly-bêta-hydroxybutyrates formant un azéotrope à minimum avec l'eau est un solvant aliphatique saturé chloré.

4. Procédé selon la revendication 3 caractérisé en ce que l'on choisit le solvant parmi les chlorométhanes, les chloroéthanes et les chloropropanes.

5. Procédé selon la revendication 4 caractérisé en ce que le solvant mis en œuvre est du 1,2-dichloroéthane.

6. Procédé selon l'une quelconque des revendications 1 à 5 caractérisé en ce que l'on élimine l'eau par voie azéotropique jusqu'à l'obtention d'un solvant anhydre.

7. Procédé selon l'une quelconque des revendications 1 à 5 caractérisé en ce que l'on élimine l'eau par voie azéotropique jusqu'à ce que le solvant des poly-bêta-hydroxybutyrates soit saturé en eau à la température considérée.

8. Procédé selon l'une quelconque des revendications 1 à 7 caractérisé en ce que la suspension aqueuse de microorganismes et le solvant d'extraction sont mis en œuvre dans des rapports en poids compris entre 1 : 2 et 1 : 50.

9. Procédé selon la revendication 8 caractérisé en ce que les rapports en poids sont compris entre 1 : 5 et 1 : 20.

4

**Claims**

1. Process for extracting poly-beta-hydroxybutyrates by means of a solvent from an aqueous suspension of microorganisms, characterised in that :

a) a solvent for poly-beta-hydroxybutyrate is chosen which forms a minimum azeotropic mixture with water,

b) the water is removed by azeotropic distillation, and

c) the extraction is carried out at least partially at the boiling point of the azeotropic mixture.

2. Process according to Claim 1, characterised in that a halogenated aliphatic to Claim 1, characterised in that a halogenated aliphatic solvent is used as a solvent for poly-beta-hydroxybutyrates which forms a minimum azeotropic mixture with water.

3. Process according to Claims 1 and 2, characterised in that the solvent for poly-beta-hydroxybutyrates which forms a minimum azeotropic mixture with water is a chlorinated saturated aliphatic solvent.

4. Process according to Claim 3, characterised in that the solvent is chosen from chloromethanes, chloroethanes and chloropropanes.

5. Process according to Claim 4, characterized in that the solvent used is 1,2-dichloroethane.

6. Process according to any one of Claims 1 to 5, characterised in that the water is removed azeotropically until an anhydrous solvent is obtained.

7. Process according to any one of Claims 1 to 5, characterised in that the water is removed azeotropically until the solvent for poly-beta-hydroxybutyrates is saturated with water at the temperature in question.

8. Process according to any one of Claims 1 to 7, characterised in that the aqueous suspension of microorganisms and the extraction solvent are used in ratios by weight of between 1 : 2 and 1 : 50.

9. Process according to Claim 8, characterised in that the ratios by weight are between 1 : 5 and 1 : 20.


**Patentansprüche**

1. Verfahren zur Extraktion von Poly-beta-hydroxybutyraten mittels eines Lösungsmittels aus einer wäßrigen Suspension von Mikroorganismen, dadurch gekennzeichnet, daß man :

a) ein Lösungsmittel für Poly-beta-hydroxybutyrat auswählt, welches ein minimales Azeotrop mit Wasser bildet,

b) das Wasser durch azeotrope Destillation entfernt und

c) die Extraktion durchführt mindestens teilweise bei der Siedetemperatur des Azeotrops.

2. Verfahren gemäß Anspruch 1, dadurch gekennzeichnet, daß man als Lösungsmittel für die Poly-beta-hydroxybutyrate welches ein minimales Azeotrop mit Wasser bildet, ein halogeniertes aliphatisches Lösungsmittel benutzt.

3. Verfahren gemäß Anspruch 1 oder 2, dadurch gekennzeichnet, daß das Lösungsmittel für die Poly-beta-hydroxybutyrate mit einem minimalen Azeotrop mit Wasser ein aliphatisches gesättigtes chloriertes Lösungsmittel ist.

4. Verfahren gemäß Anspruch 3, dadurch gekennzeichnet, daß man das Lösungsmittel auswählt unter den Chlormethanen, Chlorethanen und Chlorpropanen.

5. Verfahren gemäß Anspruch 4, dadurch gekennzeichnet, daß das eingesetzte Lösungsmittel 1,2-Dichlorethan ist.

6. Verfharen gemäß einem der Ansprüche 1 bis 5, dadurch gekennzeichnet, daß man das Wasser auf azeotropem Wege entfernt bis zur Erzielung eines wasserfreien Lösungsmittels.

7. Verfahren gemäß einem der Ansprüche 1 bis 5, dadurch gekennzeichnet, daß man das Wasser auf azeotropem Wege entfernt bis das Lösungsmittel für die Poly-beta-hydroxybutyrate mit Wasser gesättigt ist bei der betreffenden Temperatur.

8. Verfahren gemäß einem der Ansprüche 1 bis 7, dadurch gekennzeichnet, daß die wäßrige Suspension der Mikroorganismen und das Extraktionslösungsmittel eingesetzt werden in Gewichtsverhältnissen zwischen 1 : 2 und 1 : 50.

9. Verfahren gemäß Anspruch 8, dadurch gekennzeichnet, daß die Gewichtsverhältnisse zwischen 1 : 5 und 1 : 20 liegen.